# EUROPEAN PATENT APPLICATION

(11) **EP 2 494 994 A1**
(43) Date of publication of application: **05.09.2012**
(21) Application number: 12170594.1
(22) Date of filing: 26.06.2008
(51) Int. Cl.: A61L 15/26, A61L 15/42

(54) **Morselized foam for wound treatment**

(30) Priority: 29.06.2007 US 824086
(62) Divisional of application: 08771981.1
(71) Applicant: Ethicon, Incorporated, Somerville, NJ 08876 (US)
(72) Inventor: Brown, Laura J., Hamilton Square, NJ New Jersey 08690 (US); Panozzo, Mary F, Fort Wayne, IN Indiana 46814 (US)
(74) Representative: James, Anthony Christopher W.P.

(57) **Abstract**

A wound treatment material comprising morselized foam is described. The morselized foam comprises a biocompatible, biodegradable polymer. Methods for wound treatment and/or repair and/or regeneration of tissue using morselized foam are disclosed.

## Description

### FIELD

A wound treatment material comprising morselized foam for wound treatment and/or repair and/or regeneration of tissue is disclosed.

### BACKGROUND

Wounds are generally referred to as a disruption of normal anatomic structure and function, which can be present internally (underlying the skin) or externally (present on the skin surface). Wound healing is normally characterized by an orderly process through a series of distinct, but overlapping steps to culminate in wound closure (acute wounds). At times, these processes are interrupted due to multiple mechanisms or underlying pathologies (chronic wounds). Wounds vary in their location and in their duration (acute versus chronic) in addition to their underlying pathology.

Acute wounds represent approximately one tenth of the 2 billion wounds annually occurring in the US and Western Europe. Acute wounds typically heal through the body's normal healing response. Acute wounds include surgical wounds, such as those from plastic, cosmetic or reconstruction surgery, soft tissue defects, such as voids present after removal of tumors or other surgical excision, and wounds resulting from skin conditions, and traumatic injury.

Chronic wounds represent approximately 7-8 million of the 2 billion wounds that occur annually. Chronic wounds do not heal because the normal repair process of destroying damaged tissue and simultaneously forming new tissue is disrupted. Chronic wounds are delayed in their progression to closure, can remain open for months to years, and frequently reoccur. Chronic wounds may be either partial or full thickness in depth and may arise from a variety of pathological outcomes. Chronic wounds include diabetic, pressure, venous or arterial ulcers, non-healed surgical wounds, and wounds resulting from skin cancers, bums and the like.

Clinical wound assessment involves a process to define anatomic location, size, volume (depth) and undermining or tunneling as additional parameters to consider when treating wounds. Wound depth can vary from superficial, as in a partial thickness wound, which involves loss of the epidermal layer while having the dermis remain intact. Wounds may also be deep, involving not only the dermis, as in full thickness wounds, but also the underlying tissues. Wounds may present on any number of external surfaces of the body. Most commonly, wounds can occur on the extremities, in particular the feet. Wounds may occur on the toes or associated plantar surface, the heel or the ankle. All of these surfaces have very different surface topographies, which need to be considered when a clinician is applying a treatment to the surface of a wound.

Various therapies for the treatment of wounds have been described. One approach involves using tissue-engineering scaffolds. Tissue-engineering scaffolds come in a variety of forms such as weaves, knits, braids, perforated films, meshes, non-wovens, and foams. Scaffolds for tissue-engineering are utilized to provide structure and shape, to guide developing tissues, and to allow cells to attach, proliferate, and differentiate. Tissue-engineering scaffolds ("scaffolds") are typically three dimensional, highly porous structures that allow cell and tissue growth and transport of nutrients and waste. Once the newly formed tissue has filled the void, it is desirable to have the scaffold naturally degrade with minimal tissue response. The process of biodegradation can occur by enzymatic cleavage, by surface erosion or by hydrolytic cleavage. The traditional method of applying a tissue-engineering scaffold involves cutting the scaffold sheet to fit the wound and subsequently placing this scaffold into the wound bed. For deeper wounds, multiple sheets may be layered on top of one another to fill any void. The process of exact trimming of a scaffold is time consuming and varies with each patient as the particular wound or wounds may vary. Scaffold sheets are difficult to apply to topographically diverse wounds. As such, tissue-engineering scaffolds oftentimes fail to incorporate into wounds because a failure of any area of the sheet may result in expulsion of the scaffold in its entirety. This expulsion may be exacerbated by the non-uniform nature of wounds on body surfaces.

Therefore, a need still exists for a wound therapy that allows for treating wounds of a variety of sizes, shapes and depths without the need for additional manipulation prior to application and also allows for better scaffold incorporation into the wound bed by minimizing the chance for scaffold expulsion.

### SUMMARY

A morselized foam for treatment of a wound is provided. The morselized foam comprises biocompatible, biodegradable polymer, where granulation tissue overgrowth of the morselized foam at about 7 days of contact with the wound is greater than the amount of granulation tissue overgrowth for an identical non-morselized foam.

### BRIEF DESCRIPTION OF THE FIGURES

Figure 1. Photomicrograph of a histology slide depicting granulation tissue overgrowth of a morselized foam.

Figure 2. Photomicrograph of a histology slide of a foam sheet control.

### DETAILED DESCRIPTION

Compositions and methods for wound treatment and/or repair and/or regeneration of tissue using a wound treatment material comprising morselized foam, are provided. The compositions and methods allow for treating wounds of a variety of sizes, shapes and depths such that wound treatment, repair and/or regeneration of tissue is provided without the need for additional manipulation of the wound treatment material. The compositions and methods allow for improved incorporation into the wound bed by minimizing expulsion of the wound treatment material and improved granulation tissue overgrowth.

### Morselized Foam Compositions

Morselized foam is a plurality of foam pieces used to facilitate wound healing by insertion into a wound. The morselized foam may be prepared from biocompatible polymers. The biocompatible polymers used to prepare the morselized foam may also biodegradable. Biodegradable polymers may include any polymers that readily break down into small segments when exposed to moist body tissue. The segments may either be absorbed by the body, or passed by the body. Because they are absorbed by the body or passed from the body such that no permanent trace or residual of the segment is retained by the body, the biodegraded segments should not elicit permanent chronic foreign body reaction.

The morselized foam may be prepared from biocompatible, biodegradable polymers. For example, the biocompatible, biodegradable polymers may be selected from aliphatic polyesters, poly(amino acids), copoly(ether-esters), polyalkylene oxalates, polyamides, poly(iminocarbonates), polyorthoesters, polyoxaesters, polyamidoesters, polyoxaesters containing amine groups, poly(anhydrides), polyphosphazenes, and copolymer and/or blends thereof.

The morselized foam may be prepared from aliphatic polyesters. The aliphatic polyesters may include homopolymers and copolymers of lactide (which includes lactic acid, D-, L- and meso lactide), glycolide (including glycolic acid), epsilon-caprolactone, p-dioxanone (1,4-dioxan-2-one), trimethylene carbonate (1, 3-dioxan-2-one), alkyl derivatives of trimethylene carbonate, delta-valerolactone, beta-butyrolactone, gamma-butyrolactone, epsilon-decalactone, hydroxybutyrate (repeating units), hydroxyvalerate (repeating units), 1,4-dioxepan-2-one (including its dimer 1,5, 8,12-tetraoxacyclotetradecane-7,14-dione), 1,5-dioxepan-2-one, 6,6-dimethyl-1, 4-dioxan-2-one 2,5-diketomorpholine, pivalolactone, alpha, alpha-diethylpropiolactone, ethylene carbonate, ethylene oxalate, 3-methyl-1,4- dioxane-2,5-dione, 3,3-diethyl-1,4-dioxan-2,5-dione, 6,8- dioxabicycloctane-7-one, and polymer blends thereof.

The morselized foam may be prepared from biocompatible, biodegradable elastomeric copolymers. Elastomeric copolymers suitable for use as morselized foam include materials that, at room temperature, can be stretched repeatedly to at least about twice their original length and upon immediate release of the stress, return to approximately their original length. For example, suitable biocompatible, biodegradable elastomeric copolymers include, but are not limited to, copolymers of epsilon-caprolactone and glycolide (preferably having a mole ratio of epsilon-caprolactone to glycolide of from about 30/70 to about 70/30, more preferably about 35/65 to about 65/35); elastomeric copolymers of epsilon-caprolactone and lactide, including L-lactide, D-lactide blends thereof or lactic acid copolymers (preferably having a mole ratio of epsilon- caprolactone to lactide of from about 35/65 to about 65/35 and more preferably about 30/70 to about 45/55); elastomeric copolymers of p-dioxanone (1,4-dioxan-2-one) and lactide including L-lactide, D-lactide and lactic acid (preferably having a mole ratio of p-dioxanone to lactide of from about 40/60 to about 60/40); elastomeric copolymers of epsilon-caprolactone and p- dioxanone (preferably having a mole ratio of epsilon-caprolactone to p-dioxanone of from about 30/70 to about 70/30); elastomeric copolymers of p-dioxanone and trimethylene carbonate (preferably having a mole ratio of p-dioxanone to trimethylene carbonate of from about 30/70 to about 70/30); elastomeric copolymers of trimethylene carbonate and glycolide (preferably having a mole ratio of trimethylene carbonate to glycolide of from about 30/70 to about 70/30); elastomeric copolymers of trimethylene carbonate and lactide including L-lactide, D-lactide, blends thereof or lactic acid copolymers (preferably having a mole ratio of trimethylene carbonate to lactide of from about 30/70 to about 70/30) and blends thereof. In one embodiment, the elastomeric copolymer is epsilon-caprolactone and glycolide having a mole ratio of epsilon-caprolactone to glycolide of from about 30/70 to about 70/30. In another embodiment, the elastomeric copolymer is epsilon-caprolactone and glycolide having a mole ratio of epsilon-caprolactone to glycolide preferably from about 35/65 to about 65/35. In yet another embodiment, the elastomeric copolymer is 35/65 poly(epsilon-caprolactone-co-glycolide).

The morselized foams may be prepared by processes such as lyophilization, supercritical solvent foaming, for example as described in EP 464,163B1, which is incorporated by reference herein, gas injection extrusion, gas injection molding or casting with an extractable material (i.e., salts, sugar) or any other means known to those skilled in the art.

The morselized foam may be prepared by lyophilization as described in Example 2 of U.S. Patent No. 6,712,850, incorporated herein by reference in its entirety. For example, a polymer solution is prepared and poured into a mold. The mold is then transferred to a lyophilizer where the solution is frozen and then vacuum dried, thereby removing the solvent by sublimation and/or drying which results in a polymer foam sheet. The freezing step phase separates the polymer solution and the vacuum drying step removes the solvent by sublimation and/or drying, leaving a porous polymer structure or an inter-connected open cell porous foam. The porosity of the morselized foam is preferably greater than 90 percent by volume. The morselized foam thickness is preferably from about 0.25 mm to about 0.75 mm. More preferably, the thickness of the morselized foam is from about 0.4 mm to about 0.6 mm.

The morselized foam may be prepared in the form of a sheet or other geometrical shape of large size which is later reduced to smaller pieces as described below. The features of the morselized foams may be controlled to suit the desired application, for example, by a modified lyophilization process that results in (1) interconnecting pores of sizes ranging from 10 to 200 microns (or greater) that provide pathways for cellular ingrowth and nutrient diffusion; (2) porosities preferably ranging from 90% or higher; and (3) channels that run through thickness of the morselized foam for improved vascularization and nutrient diffusion.

The morselized foam may be prepared by cutting the aforementioned large sheets or other geometric shapes into pieces or by creating individual morselized foam pieces by molding, extrusion, or other methods of creating foams known to those of ordinary skill in the art. The morselized foam is cut or formed into pieces of dimensions adapted for optimal performance as a wound treatment material. A majority of the foam pieces preferably have a porosity of greater than 90 percent by volume, a thickness of about 0.25 mm to about 0.75 mm, a length of about 1 mm to about 4 mm, and a width of about 1 mm to about 4 mm. For example, the length of the pieces may independently be from about 1 mm to about 4 mm and the width of the pieces may Independently be from about 1 mm to about 4 mm and a thickness of about 0.25 mm to about 0.75 mm. Other sizes may be used. A majority of foam pieces refers to more than 50% of the total volume of the wound treatment material. Preferably, a majority of foam pieces would be more than 75% of the total volume of the wound treatment material. More preferably, a majority of foam pieces would be more than 90% of the total volume of the wound treatment material. The pieces may be of a uniform shape, a non-uniform shape, or a mixture of pieces of uniform and non-uniform shape. The morselized foam may be of any regular or irregular polygon shape or mixtures of such shapes. For example, the morselized foam may be substantially square in shape. The foam sheet or other geometric shape may be morselized using mechanical equipment or may be morselized by hand. The morselized foam may be sieved, sorted, and/or separated such as to provide morselized foam of a predetermined shape and/or within a predetermined size or range of sizes. The range of sizes of the pieces may be represented by a distribution having a mean and/or average size calculated by standard statistical methods. For example, the mean or average size of morselized foam pieces may be from about 1 mm to about 4 mm.

### Methods of Wound Treatment and/or Repair and/or Regeneration of Tissue

A method of tissue repair and regeneration is provided. The method comprises contacting a wound of a subject with a wound treatment material. The wound treatment material comprises biocompatible, biodegradable morselized foam. Other wound treating materials may be used in combination or concurrently with the wound treatment material herein disclosed. For example, the morselized foam may be dispersed or suspended in a therapeutically acceptable carrier such as a gel, cream, or ointment, for application to a wound site.

The wound treatment material may be used for wound treatment and/or the repair and/or the regeneration of tissue. The wounds may be acute or chronic wounds of the skin or tissue. Acute wounds include surgical wounds, such as those from plastic, cosmetic or reconstruction surgery, soft tissue defects, such as voids present after removal of tumors or other surgical excision, wounds resulting from skin conditions, and traumatic injury. Chronic wounds may be either partial or full thickness in depth and may arise from a variety of pathological outcomes. Chronic wounds include diabetic, pressure, venous or arterial ulcers, non-healed surgical wounds, and wounds resulting from skin cancers, bums, and the like.

The morselized foam may provide for treating wounds of a variety of sizes, shapes and depths without the need for additional manipulation of the wound treatment material prior to application. Thus, a healthcare provider would not be required to modify the wound treatment material, for example, by cutting to size or shape, prior to application to the wound site.

When the morselized foam as herein disclosed is incorporated as a tissue-engineering scaffold, scaffold expulsion is minimized or prevented. Typically, when applying a tissue engineering scaffold to deep wounds or wounds with limited blood flow, cell migration into the scaffold is limited to the rate at which a cell can invade. A tissue engineering scaffold of morselized foam as herein described, which exists as multiple pieces, may increase cell migration into the interstices of the scaffold in a manner such as to facilitate faster cell migration and/or incorporation.

Morselized foams used as tissue scaffolds preferably possess a structure that provides organization at the microstructural level and/or a template that facilitates cellular organization that mimics natural tissue, such as dermal tissue. The cells will adhere, proliferate and differentiate along the contours of the morselized foam pieces. This may result in a tissue that substantially mimics the anatomical features of tissue, for example, dermal tissue.

The morselized foam when introduced to a wound site, preferably results in more than 50% of the morselized foam pieces being infiltrated and/or covered by granulation tissue within about 7 days. More preferably, about 75% to 90% of the morselized foam pieces are infiltrated and/or covered by granulation tissue within about 7 days. Even more preferably, substantially all of the morselized foam pieces are infiltrated and/or covered by granulation tissue within about 7 days after introduction to a wound site. Measurements of infiltrated granulation tissue may be obtained from histological slides using optical microscopic techniques and/or image analysis techniques.

Granulation tissue overgrowth is measured in either of two ways by analysis of histological slides of the wound site. The minimum distance (average ± SEM (mm)) from the top of the morselized foam to the surface of the wound site is calculated. Or, the maximum distance (average ± SEM (mm)) from the top of the morselized foam to the surface of the wound site is calculated.

Granulation tissue overgrowth of the wound treatment material, for example, of one or more of the morselized foam pieces, as measured by optical microscopy of histological sections of the wound after 7 days, is greater than the amount of tissue overgrowth from identical non-morselized foam. An "identical non-morselized foam" is prepared in exactly the same way as the morselized foam except it is a single sheet (or other geometrical shape). The amount of granulation tissue overgrowth of the morselized foam is greater than an identical non-morselized foam if either of the above described tissue overgrowth distances are greater for the morselized foam than the non-morselized foam.

The morselized foam may contain or be impregnated, encapsulated or coated with one or more agents capable of stimulating or enhancing the attachment, proliferation or differentiation of tissue, such as granulation tissue. Such agents may include anti-infectives, hormones, analgesics, anti-inflammatory agents, growth factors, chemotherapeutic agents, anti-rejection agents, prostaglandins, and RGD peptides. The morselized foam may contain or be impregnated, encapsulated or coated with one or more postpartum cells, cell products, or cell derivatives derived from umbilical cord tissue as described in US Patent Application Publication No. 2005/005498, or from postpartum cells, cell products, or cell derivatives derived from placental tissue, as described in US Patent Application Publication No. 2005/0058631, the disclosures of which are incorporated herein by reference. The morselized foam may contain or be impregnated, encapsulated or coated with one or more cell products, cell derivatives, myocytes, adipocytes, fibromyoblasts, ectodermal cell, muscle cells, osteoblast, chondrocyte, endothelial cells, fibroblast, pancreatic cells, hepatocyte, bile duct cells, bone marrow cells, neural cells, genitourinary cells, pluripotent cells, stem cells, precursor cells, and cell products or cell derivatives thereof.

The wound treatment material comprising morselized foam as herein disclosed may be sterilized by conventional sterilization techniques. For example, the wound treatment material comprising morselized foam may be packaged and sterilized and provided ready for use.

The following examples are illustrative and not to be interpreted as limiting or restrictive. Notwithstanding that the numerical ranges and parameters setting forth the broad scope of the invention are approximations, the numerical values set forth in the specific examples are reported as precisely as possible. Any numerical value, however, inherently contain certain errors necessarily resulting from the standard deviation found in their respective measurements. For example, the use of the term "about" in reference to a numerical value refers to a range of approximately ± 10 percent unless specified otherwise.

### Example 1A: Preparation of morselized foam and controls

A 5 weight/volume percent solution of 35/65 mole/mole percent poly (epsilon-caprolactone-co-glycolic acid) (PCL/PGA) copolymer (Ethicon, Inc., Somerville, NJ) 1,4-dioxane (Fisher Chemical, Fairlawn, NJ) was prepared by adding one part polymer to every nine parts of solvent. The polymer and solvent were heated with stirring at 60 degrees Celsius for approximately 4-8 hours until the polymer dissolved. The polymer solution was filtered and about 10 grams of the solution was poured into an aluminum mold approximately 4.5 inches (114millimeters) square and approximately12.7 millimeters deep. The polymer solution filled molds were placed on the shelf of an FTS Dura Dry Freeze dryer that was precooled to -17°C. After 15 minutes the cycle was started and the temperature was held at -17°C for 60 minutes. Vacuum was then applied to initiate primary drying of the dioxane by sublimation at 100 mTorr for 60 minutes. Next secondary drying was conducted a 5°C for 60 minutes and 20°C for 60 minutes. The vacuum was maintained at 20 mTorr. Finally, the lyophilizer was brought to room temperature and vacuum was broken with dry nitrogen. After purging with dry nitrogen for 30 minutes the door was opened and foam was lifted from the mold. Foams prepared accordingly may be about 0.5 mm in thickness and may be of a porosity greater than 90%, which may be determined, for example, by Helium Pycnometry, per ASTM standard test method D6226, "Open cell contents of rigid cellular plastics". *In vivo* studies of such foams may demonstrate that the foam would be completely absorbed by the body within about 90 to 120 days.

### Example 1B

The morselized foam was prepared as follows. The foam prepared as described in Example 1A was cut into 1.5 x 1.5 centimeter sheets. 12 foam sheets were prepared in a similar manner and individually pouched and sterilized using gamma irradiation. These foam sheets were used as controls. Morselized foam samples were prepared by cutting 6 of the sterile 1.5 x 1.5 cm foam sheets into approximately 1-4 mm pieces immediately prior to placing in the wound bed.

### Example 2: Pig Tissue Repair and Regeneration Model

The purpose of this study was to determine the cellular infiltration and wound healing response of morselized foam in a full-thickness excisional defect.

### Experimental Design:

Two treatment groups were prepared for comparison. The first treatment group consisted of morselized foam as prepared in EXAMPLE 1B. The second treatment group consisted of the foam sheet controls. Full thickness excisional wounds (approximately 1.5 x 1.5 centimeter square) were created on the dorsal region of four swine. The treatment groups were randomized over the pigs and left in place throughout the study period. There was an n of 6 per treatment group.

The foam sheet controls were trimmed to fit the wound and placed in the wound bed. Morselized foam pieces (approximately 13-35) were placed in the wound bed. Each wound was covered with a 2 x 2 centimeter wound dressing sold under the tradename NU-GEL (Johnson and Johnson Medical, Arlington, TX), followed by covering with a transparent wound dressing sold under the tradename BIOCLUSIVE (Johnson and Johnson Medical, Arlington, TX). Strips of self-adhering foam, sold under the trade name RESTON (3M Medical Surgical Division, St. Paul, Minnesota), were placed between sites to prevent cross-contamination due to wound fluid leakage. Sterile gauze was secured over the dorsum of the back with a plaster tape sold under the tradename ZONAS (Johnson and Johnson Medical, Arlington, TX). A body stockinet sold under the tradename SPANDAGE (Medi-Tech International Corporation, Brooklyn, New York) was used to hold the dressings in place. On day, 2 post-wounding, the bandages were changed.

Tissues were harvested from the animals on day 7. The entire wound and surrounding normal skin was excised and placed in 10% neutral buffered formalin. The tissue was bisected and the cranial half was sent for histological processing.

### Histological Assessments:

Sections were analyzed for amount of granulation tissue overgrowth (measured by depth of the scaffold within the wound bed) and incorporation of the control or morselized foam into the wound bed. The amount of tissue overgrowth for each histological slide was determined using two evaluations. In the first evaluation, the minimum distance from the top of the control or morselized foam piece to the surface of the wound was calculated. In the second evaluation, the maximum distance from the top of the control or morselized foam piece to the surface of the wound was calculated. The results of these measurements are summarized in Table 1. The histological slides were also evaluated for scaffold extrusion, which is a visual determination of whether any portion of the scaffold has been pushed out or extruded from the wound.

| TABLE 1. | | |
|---|---|---|
| Treatment | Minimum Distance from top of scaffold to wound surface (Average ± SEM) (mm) | Maximum Distance from top of scaffold to wound surface (Average ± SEM) (mm) |
| Control (foam sheet) | 0.00 ± 0.00 | 1.44 ± 0.42 |
| Morselized foam pieces | 0.33 ± 0.13 | 2.67 ± 0.38 |

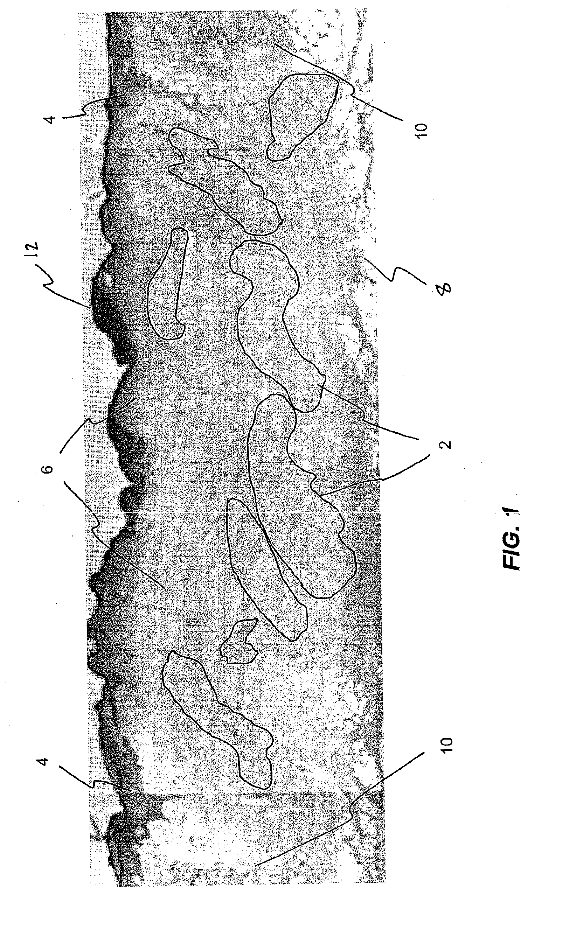

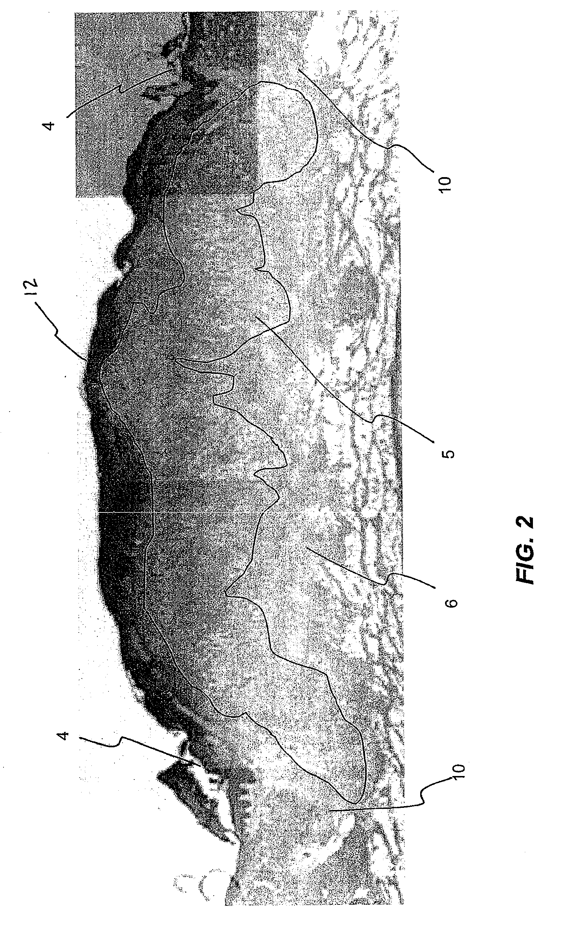

Referring now to the FIGURES, representative histology slides depicting the difference in incorporation into the wound bed between the morselized foam (FIG. 1) and foam sheet control (FIG. 2) are provided. FIG. 1 depicts a wound defined by wound edges 10, wound bed 8, and wound surface 12 containing incorporated morselized foam pieces 2 within granulation tissue 6 and beneath epithelial tongue 4. FIG. 2 depicts a wound defined by wound edges 10, wound bed 8, and wound surface 12 containing control 5 beneath wound surface 12 and beneath epithelial tongue 4.

At least one portion of the control was at the surface of the wound in each of the six test sites, for example, as shown in FIG. 2, (average minimum distance of 0 mm from the top surface of the wound). In contrast, the morselized foam averaged a minimum distance of 0.33 mm from the top surface of the wound. The top of the control averaged 1.44 mm at its deepest in the wound while the morselized foam averaged 2.67 mm at its deepest in the wound. The foam pieces were consistently deeper in the wound with more overlying granulation tissue than the controls. In half of the six treatments, the control was at least partially extruded from the wound bed. In contrast, the majority of morselized foam pieces were deeply incorporated into the wound bed with only a few pieces of morselized foam near the wound surface.

Although a few pieces of a morselized foam may extrude from the wound, many other pieces remain to benefit the tissue repair and regeneration process. In contrast, if part of the foam sheet control is extruded from a part of the wound, the foam sheet as a whole may be extruded. Moreover, if part of the foam sheet is extruded from a part of the wound that portion of the foam sheet will not be available as a scaffold.

The histology section depicted in FIG. 1 shows the morselized foam deep in the wound bed and covered by and infiltrated by granulation tissue at day 7, whereas the control is predominately at the wound surface at day 7, as shown in FIG. 2, demonstrating that the morselized foam incorporated into the wound bed faster than the foam sheet control.

### Example 3: Clinical Application of Morselized Foam for Tissue Repair and Regeneration

Patients in need of tissue repair and regeneration who would be treated with the morselized foam may have the foam pieces directly applied to the wound without further pretreatment of the wound site. For some chronic wounds, it may be necessary to prepare the wound site for incorporation of the morselized wound treatment material. This may be accomplished by surgical, mechanical, chemical, autolytic methods, maggot therapy, or combinations thereof.

Necrotic or non-viable tissue within or adjacent the wound site may be removed using a variety of methods. For example, the wound site may be prepared by debridement. For debridement of the wound site, local anesthetic may be applied as appropriate. For surgical debridement, a sharp instrument, scalpel, curette or laser can be used to remove large amounts of necrotic tissue especially when infection is associated with the wound. Mechanical debridement to remove dead tissue, which offers a low cost means of debridement, may be performed using gauze. Because this approach does not require surgical skills, it may be easily accomplished by a nurse in a wound care setting.

Wounds, either debrided or non-debrided, may be first washed with sterile saline prior to application of the morselized foam wound treatment material. The morselized foam wound treatment material may be applied to a clean wound, which is free of clinical signs of infection or may be directly applied to a wound without preparation. The number of morselized foam wound treatment material pieces applied to the wound may be determined by the clinician, taking into account the wound size including area and thickness. The morselized foam wound treatment material may then be subsequently covered with a non-adherent secondary dressing and/or appropriate other wound dressings depending on the wound type and location.

### Occupied Total Wound Volume Comparison of Morselized Foam with Control

The measured wound depth of the full thickness excisional wounds (approximately 1.5 cm x 1.5 cm) created on the dorsal region of the four swine ranged from 2.21-2.36 mm. Therefore, the calculated total wound volume is approximately 515 mm³. The 1.5 cm x 1.5 cm foam sheet control of about 0.5 mm thickness placed in the wound was calculated to have a volume of 113 mm³, which was calculated to occupy approximately 22% of the total wound volume.

Approximately 35 morselized foam pieces of 1-2 mm average length would occupy about 7.5% of the same 515 mm³ wound volume. Approximately 13 morselized foam pieces of 3-4 mm average length would occupy about 15.5% of the same 515 mm³ wound volume. Therefore, the average morselized foam piece occupies approximately 0.2% to about 1.2% of the total wound volume for Example 2. Specific average sized morselized foam pieces or ranges of different average sized morselized foam pieces may be used to provide for occupying a desired wound volume. Preferably, the average morselized foam piece occupies approximately 0.1 % to about 5% of the total wound volume.

As used herein, "comprising," "including," "containing," "characterized by," and grammatical equivalents thereof are inclusive or open-ended terms that do not exclude additional, unrecited elements or method steps. "Comprising" is to be interpreted as including the more restrictive terms "consisting of" and "consisting essentially of."

As used herein, "consisting of" and grammatical equivalents thereof exclude any element, step, or ingredient not specified in the claim.

As used herein, "consisting essentially of" and grammatical equivalents thereof limit the scope of a claim to the specified materials or steps and those that do not materially affect the basic and novel characteristic or characteristics of the claimed invention.

Other embodiments within the scope of the claims herein will be apparent to one skilled in the art from consideration of the specification or practice of the invention as disclosed herein. It is intended that the specification, together with the examples, be considered to be exemplary only, with the scope and spirit of the invention being indicated by the claims.

The invention also encompasses the following embodiments
1. A morselized foam treatment of a wound, the morselized foam comprising biocompatible, biodegradable polymer wherein granulation tissue overgrowth of the morselized foam at about 7 days of contact with the wound is greater than the amount of granulation tissue overgrowth for an identical non-morselized foam.
2. The morselized foam of embodiment 1 wherein the biocompatible, biodegradeable polymer is selected from the group consisting of aliphatic polyesters, poly(amino acids), copoly(ether-esters), polyalkylene oxalates, polyamides, poly(iminocarbonates), polyorthoesters, polyoxaesters, polyamidoesters, polyoxaesters containing amine groups, poly(anhydrides), polyphosphazenes, and combinations thereof.
3. The morselized foam of embodiment 2 wherein the aliphatic polyester is homopolymers and copolymers of monomers selected from the group consisting of lactide, glycolide, epsilon-caprolactone, p-dioxanone, trimethylene carbonate and combinations thereof.
4. The morselized foam of embodiment 2 wherein the aliphatic polyester is an elastomeric copolymer selected from the group consisting of poly(epsilon-caprolactone-co-glycolide), poly(epsilon-caprolactone-co-lactide), poly(p-dioxanone-co-lactide; poly(epsilon-caprolactone-co-p-dioxanone); poly(p-dioxanone-co-trimethylene carbonate); poly(trimethylene carbonate-co-glycolide); poly(trimethylene carbonate-co-lactide) and combinations thereof.
5. The morselized foam of embodiment 4 wherein the elastomeric copolymer is poly(epsilon-caprolactone-co-glycolide) having a mole ratio of epsilon-caprolactone to glycolide of from about 30/70 to about 70/30.
6. The morselized foam of embodiment 4 wherein the elastomeric copolymer is poly(epsilon-caprolactone-co-glycolide) having a mole ratio of epsilon-caprolactone to glycolide of from about 35/65 to about 65/35.
7. The morselized foam of embodiment 4 wherein the elastomeric copolymer is poly(epsilon-caprolactone-co-glycolide) having a mole ratio of epsilon-caprolactone to glycolide of 35/65.
8. The morselized foam of embodiment 1 wherein the foam has a porosity of greater than 90 percent by volume
9. The morselized foam of embodiment 1 wherein the foam has a thickness of about 0.25 mm to about 0.75 mm.
10. The morselized foam of embodiment 1 wherein the foam has a width of about 1 mm to about 4 mm and a length of about 1 mm to about 4 mm.
11. The morselized foam of embodiment 1 wherein the foam has a porosity of greater than 90 percent by volume, a thickness of about 0.25 mm to about 0.75 mm, a width of about 1 mm to about 4 mm and a length of about 1 mm to about 4 mm..
12. The morselized foam of embodiment 1, wherein the average piece of morselized foam occupies about 0.1% to about 5% of the total wound volume.
13. A morselized foam comprising a biocompatible, biodegradable elastomeric copolymer of poly(epsilon-caprolactone-co-glycolide), the copolymer having a mole ratio of epsilon-caprolactone to glycolide of 35/65;
   wherein the morselized foam has a porosity of greater than 90 percent by volume, a thickness of about 0.25 mm to about 0.75 mm, a width of about 1 mm to about 4 mm, and a length of about 1 mm to about 4 mm.
14. The morselized foam of embodiment 1, further comprising one or more agents capable of stimulating or enhancing the attachment, proliferation or differentiation of tissue, the one or more agents being impregnated, encapsulated and/or coated thereon and/or therein.
15. The morselized foam of embodiment 14, wherein the one or more agents are selected from:
   (i) anti-infectives, hormones, analgesics, anti-inflammatory agents, growth factors, chemotherapeutic agents, anti-rejection agents, prostaglandins, RGD peptides;
   (ii) postpartum cells derived from umbilical cord tissue, postpartum cells derived from placental tissue, or their cell products or derivatives; and
   (iii) myocytes, adipocytes, fibromyoblasts, ectodermal cell, muscle cells, osteoblast, chondrocyte, endothelial cells, fibroblast, pancreatic cells, hepatocyte, bile duct cells, bone marrow cells, neural cells, genitourinary cells, pluripotent cells, stem cells, precursor cells, or their cell products or derivatives thereof.
16. A method of tissue repair and/or regeneration, the method comprising:
   contacting a wound of a subject with morselized foam comprising biocompatible, biodegradable polymer wherein granulation tissue overgrowth of the morselized foam at about 7 days of contact with the wound is greater than the amount of granulation tissue overgrowth for an identical non-morselized foam.
17. The method of embodiment 16, wherein the morselized foam comprises a porosity of greater than 90 percent by volume, a thickness of about 0.25 mm to about 0.75 mm, a length of about 1 mm to about 4 mm, and a width of about 1 mm to about 4 mm.
18. The method of embodiment 17, wherein further comprising one or more agents capable of stimulating or enhancing the attachment, proliferation or differentiation of tissue, the one or more agents being impregnated, encapsulated and/or coated thereon and/or therein.
19. The method of embodiment 18, wherein the one or more agents are selected from:
   (i) anti-infectives, hormones, analgesics, anti-inflammatory agents, growth factors, chemotherapeutic agents, anti-rejection agents, prostaglandins, RGD peptides;
   (ii) postpartum cells derived from umbilical cord tissue, postpartum cells derived from placental tissue, or their cell products or derivatives; and
   (iii) myocytes, adipocytes, fibromyoblasts, ectodermal cell, muscle cells, osteoblast, chondrocyte, endothelial cells, fibroblast, pancreatic cells, hepatocyte, bile duct cells, bone marrow cells, neural cells, genitourinary cells, pluripotent cells, stem cells, precursor cells, or their cell products or derivatives thereof.
20. The method of embodiment 16, wherein the average piece of morselized foam occupies about 0.1% to about 5% of the total wound volume.

## Claims

1. A morselized foam comprising a biocompatible, biodegradable elastomeric copolymer of poly(epsilon-caprolactone-co-glycolide), the copolymer having a mole ratio of epsilon-caprolactone to glycolide of 35/65; wherein the morselized foam has a porosity of greater than 90 percent by volume, a thickness of about 0.25 mm to about 0.75 mm, a width of about 1 mm to about 4 mm, and a length of about 1 mm to about 4 mm.

2. The morselized foam of claim 1, further comprising one or more agents capable of stimulating or enhancing the attachment, proliferation or differentiation of tissue, the one or more agents being impregnated, encapsulated and/or coated thereon and/or therein.

3. The morselized foam of claim 2, wherein the one or more agents are selected from:
(i) anti-infectives, hormones, analgesics, anti-inflammatory agents, growth factors, chemotherapeutic agents, anti -rejection agents, prostaglandins, RGD peptides;
(ii) postpartum cells derived from umbilical cord tissue, postpartum cells derived from placental tissue, or their cell products or derivatives; and
(iii) myocytes, adipocytes, fibromyoblasts, ectodermal cell, muscle cells, osteoblast, chondrocyte, endothelial cells, fibroblast, pancreatic cells, hepatocyte, bile duct cells, bone marrow cells, neural cells, genitourinary cells, pluripotent cells, stem cells, precursor cells, or their cell products or derivatives thereof.

4. A morselized foam, as described in any one of claims 1 to 3, for use in a method of tissue repair and/or regeneration, the method comprising contacting a wound of a subject with the morselized foam, wherein granulation tissue overgrowth of the morselized foam at 7 days of contact with the wound is greater than the amount of granulation tissue overgrowth for an identical non-morselized foam.
